# EUROPEAN PATENT APPLICATION

(11) **EP 3 909 512 A1**
(43) Date of publication of application: **17.11.2021**
(21) Application number: 21173517.0
(22) Date of filing: 12.05.2021
(51) Int. Cl.: A61B 8/08, A61B 8/00, A61N 7/00

(54) **DETECTION OF TUMORS USING ULTRASOUND**

(30) Priority: 12.05.2020 US 202063023757 P; 07.05.2021 US 202117314808
(71) Applicant: Acoustiic Inc., Pacific Palisades, California 90272 (US)
(72) Inventor: Reynolds, Paul, Pacific Palisades 90272 (US); Taffler, Sean, Pacific Palisades 90272 (US)
(74) Representative: Diehl & Partner

(57) **Abstract**

Techniques are provided for detection of tumors using ultrasound. An early detection test may be performed on a patient. A location of a tumor may be determined based on the early detection test. Properties of the tumor may be determined based on the early detection test. Moieties may be functionalized based on the properties of the tumor. The moieties maybe introduced into the patient. The location of the tumor may be imaged using ultrasound, magnetic resonance elastography, or computed tomography to generate images of the location of the tumor. A treatment plan based on the images of the location of the tumor may be implemented using ultrasound.

## Description

### BACKGROUND

Methods of detecting the presence of cancer in the body quickly and at relatively low cost are becoming more prevalent. For example, free circulating DNA or other detection modalities may allow for early detection of tumors prior to clinical significance. The DNA or otherwise may give an indication of the type of tumor, and therefore of the potential location of the tumor within the body. These methods may be limited in the accuracy with which they can locate the tumor within the body. For example, a blood test may show that a tumor is "in the body" or "in the liver". A screening imaging test may not accurately locate all tumor sites, as a screening imaging test may find the cancer as a byproduct of another scan. It may be difficult to locate a tumor exactly based on rough information provided by many quick and low-cost methods of detection, such as a free circulating DNA that indicates that the liver as the primary organ of origin of a tumor. Even if a tumor is located, at small sizes it may often not be appropriate to perform major surgery, chemotherapy, radiation therapy, or the like, as these treatments may be more damaging, and costly, than the tumor itself at the time the tumor is detected.

Imaging modalities such as magnetic resonance (MR) may be used to locate tumors via imaging and automated analysis. However, the time and computational resources needed to conduct a full body scan using MR are significant. Other imaging modalities, such as MR Elastography, to detect stiffness of tissue inhomogeneities, which correlates to cancer likelihood, may be difficult to perform on the whole body. These imaging modalities may work well when the search region can be limited. Ultrasound waves may be used to provide the stimulus, or push pulse, for MR elastography. It may be possible to undertake elastography using only an ultrasound system, thereby removing the reliance on an MR system. Ultrasound Elastography may be faster, more accurate, and higher resolution than MR, however there may be no equivalent 3D volumetric equivalent system for Ultrasound Elastography.

### BRIEF SUMMARY

According to implementations of the disclosed subject matter, an early detection test may be performed on a patient. A location of a tumor may be determined based on the early detection test. Properties of the tumor may be determined based on the early detection test. Moieties may be functionalized based on the properties of the tumor. The moieties maybe introduced into the patient. The location of the tumor may be imaged using one or more of ultrasound, magnetic resonance elastography, and computed tomography elastography to generate images of the location of the tumor. A treatment plan based on the images of the location of the tumor may be implemented using ultrasound.

Additional features, advantages, and implementations of the disclosed subject matter may be set forth or apparent from consideration of the following detailed description, drawings, and claims. Moreover, it is to be understood that both the foregoing summary and the following detailed description provide examples of implementations and are intended to provide further explanation without limiting the scope of the claims.

### BRIEF DESCRIPTION OF THE DRAWINGS

The accompanying drawings, which are included to provide a further understanding of the disclosed subject matter, are incorporated in and constitute a part of this specification. The drawings also illustrate implementations of the disclosed subject matter and together with the detailed description serve to explain the principles of implementations of the disclosed subject matter. No attempt is made to show structural details in more detail than may be necessary for a fundamental understanding of the disclosed subject matter and various ways in which it may be practiced.
FIG. 1 shows an example arrangement for detection and treatment of tumor using ultrasound.
FIG. 2 shows an example arrangement for detection and treatment of tumor using ultrasound.
FIG. 3 shows an example procedure for detection and treatment of tumor using ultrasound.
FIG. 4 shows a computer according to an embodiment of the disclosed subject matter.
FIG. 5 shows a network configuration according to an embodiment of the disclosed subject matter.

### DETAILED DESCRIPTION

Using pre-existing information such as blood markers, free circulating DNA, and/or byproduct scans and tests, the search space used when searching for a tumor in a body of a patient may be narrowed. Moieties with an affinity for tumors, or that aggregate at tumor sites, may be introduced into the body of the patient based on the narrowed search space and the information already gathered about the tumor. The moieties may be engineered to be detectable by an intended imaging modality that will be used to image the location corresponding to the narrowed searched space. Labeling of a tumor using moieties based on the affinity of the moieties for the tumor may allow for the tumor to be more accurately located within the body, particularly when locating small tumors that are at the imaging resolution of the utilized imaging modality. Enhanced accuracy in the location of the tumor may allow for subsequent treatment to be delivered with greater accuracy and precision, only treating that specific area with minimized damage margins because of the increased ability to accurately and precisely locate the tumor. The imaging modality used to locate a tumor and therapy system used to treat the tumor may or may not use the same imaging method. It may also be possible to use the same ultrasound system as both an imaging system and therapy system. The imaging system and the ultrasound system may be separate but coincident in the ultrasound system. Regardless of the imaging modality used to locate the tumor, the imaging system and therapy system may act either simultaneously, or within a very short timeframe, and without either the patient or either the imaging system or therapy system moving, allowing for increased accuracy and precision in delivering therapy to the location of the tumor.

The moieties may be tags, or contrast agents, which may have an affinity for tumors and may provide contrast for visibility in images generated by the various imaging modalities. The moieties may be optimized for the imaging modality being used to locate a tumor. For example, in an MR system the moieties may have a marker that may be visible via MR imaging, such as gadolinium. If an ultrasound system is used for imagining, the moieties may be microbubbles or nanobubbles. The microbubbles or nanobubbles may be filled with a suitable perfluourinated compound. This may allow the microbubbles or nanobubbles to get inside the cells of the tumor. A frequency of ultrasound that causes the micro or nanobubbles to heat, expand, oscillate, and/or burst may be applied to the general area of the tumor. This may allow imaging ultrasound frequencies to be used to detect the heated, expanded, oscillating, or burst microbubbles or nanobubbles in the cells of the tumor, allowing the cells of the tumor to be located in images generated through ultrasound imaging.

The moieties may allow for therapy planning for treatment for all locations identified through the moieties' affinity for tumors. The affinity of moieties to the tumor may be based on gross blood flow, for example, based on increased vascularization due to angiogenesis of the tumor, or the moieties may be functionalized to attach to surface proteins or other features of the tumor cells. The functionalizing of the moieties may use the surface protein structure of the cells of the tumor, cell surface protein (CSP). The CSP of a tumor may be found either through wet chemistry of the target tumor cells or through cross referencing an extant database that correlates tumors and the CSPs based on, for example, the organ in which the tumor is located or other properties of the tumor determined, for example, through early detection tests. The moieties may be functionalized through the use of common streptavidin and biotin complex, or through any other suitable chemistry that may adhere protein or chemical tags to the moieties and cause the moieties to have an affinity for a tumor of the type that the moieties are intended to target.

In an ultrasound system, the moieties may also provide information about the path between the target, for example, the tumor, and the transducer array of the ultrasound system. A nonlinear effect from the micro or nanobubbles may cause them to radiate energy in different frequency bands. These frequency bands may be used to estimate tissue properties of tissue between the target, for example, the tumor, and the transducer array, and thus improve resolution and performance of the ultrasound system as a whole.

To detect and treat a tumor, an early detection test, such as a blood test or a histology test, may first be used to tell there is cancer in a patient's body, and to determine the likely organ of origin of the cancer, which may be the organ in which a tumor is located. The DNA or other data gathered through the blood test, or other early detection test, may give an indication of the type of tumor, and therefore of the potential location of the tumor within the body, for example, the organ in which the tumor is likely to be located. The best way to functionalize moieties, or contrast agents, for the desired imaging modality, may be determined based on the results of the blood test or other early detection test. For example, the moieties may be functionalized by using the results of the blood test to identify the CSPs on cells from a tumor using wet chemistry or to cross reference an extant database that correlates tumors and the CSPs, for example, based on the organ in which the tumor is located. After the introduction of the functionalized moieties into the patient, the organ where the tumor is located may be imaged using any suitable technique, such as, for example, contrast, elastography, or attenuation imaging. Data resulting from the imaging of the organ may be analyzed and presented to a doctor, who may determine an appropriate treatment plan. In some implementations, the data resulting from the imaging may be input to an artificial intelligence system or expert system that may use the data to suggest an appropriate treatment plan. The treatment plan may identify specific locations in the organ in which the tumor is located to which treatment should be applied. The moieties may be visible in the images generated from the imaging of the organ, and the moieties locations in the image may correspond to the location of the tumor, or cells from the tumor, within the organ, and may be locations where treatment should be applied. Focused ultrasound may be applied to the specific locations in the organ identified by the treatment plan, using the moieties as a guide to ensure accuracy and precision in the targeting of the ultrasound waves. After treatment, the patient may return for additional blood tests and scans to determine if further treatment is needed.

Early detection of tumors, using blood tests that detect tumors through free circulating DNA or using other types of detection, such as histology, may locate the tumor as being in a particular organ, but may not locate the tumor precisely within the organ. A tumor may be small, for example, having a volume of only a few cubic millimeters, while the organ the tumor is in may be much larger, making the tumor more difficult to locate within the organ. Widespread use of blood tests and other types of early detection for tumors may result in a larger number of tumors being detected, in turn resulting in a larger number of patients who will need imaging in order to locate the tumor. These patients may reside in locations without large hospitals or clinics nearby. In order to be effective for such patients, the ultrasound system for imaging and treatment may need to be able to image large volumes, for example, organs such as the liver, quickly in 3-dimensions, have sufficient resolution to detect small tumors, be low cost and small enough to be portable or available to smaller clinics, be non-ionizing to be safe to use repeatedly and in large scale, have minimal to no contraindications for use in a wide population, be able to combine imaging and therapy in the same system or in the same patient visit, work rapidly enough that multiple patients can be imaged and treated in the same day, be able to monitor the effects of therapy both during and following the treatment, and be usable for most to all locations within the body.

MR may be too large and costly with contraindications and may not be able to perform therapy. Computed Tomography may be costly and may use ionizing radiation and may not be able to perform therapy. Optical and laser methods may only be useful for surface or close to surface masses. There are no existing ultrasound systems which may be able to measure 3D volumes of objects on the same scale as a liver, nor may there be any 3D volume imagers which can perform therapeutic ultrasound surgery.

An ultrasound system for the detection and treatment of tumors may have a transducer array with a very large area, for example, greater than 10 centimeters (cm)², as compared to most ultrasound devices, which may between 2 cm² to 10 cm². The active area of the transducer array may have a surface area of greater than 15 cm². The ultrasound system may have an elevation larger than most ultrasound devices, for example, greater than 2cm, and an azimuth larger than most ultrasound devices, for example, greater than 5cm. For example, the ultrasound system of detection and treatment of tumors may have a transducer array that is 10 cm by 10 cm, as compared to transducer arrays of the largest currently standard devices, which may be 0.6 cm by 5 cm, or 2.4 cm by 5 cm. The transducer array of the ultrasound system may also be fully sampled in both directions, for example, at least lambda / 2, with no missing elements, a matrix transducer array, as compared to other ultrasound devices which may only be well sampled in azimuth. Because of this, the ultrasound system for detection and treatment of tumors may be able to, when operating as an imaging system, generate images that are closer to those generated by MR, for example, a 3D volume image, rather than live scan slices that have difficulty either locating targets exactly or ensuring an exact slice The usual limitation in voxel size in conventional transducers is the frequency of operation. Because the ultrasound system has a transducer array that is a matrix transducer array with a large area, the ultrasound system may be able to image a 'voxel' smaller in volume than traditional ultrasound systems operating at the same frequency. The transducer array of the ultrasound system may include any suitable number of ultrasonic transducer elements of any suitable types, arranged in any suitable manner to form the transducer array.

The ultrasound system for detection and treatment of tumors may be able to operate in a therapy mode to provide the acoustic power levels and accurate focusing to perform the treatments of tumors located though imaging. For small tumors, combined imaging and therapy may take only a few minutes. The ultrasound system, combining an imaging system and therapy system, may be able to monitor the patient both during the treatment and during follow-up visits. The ultrasound system may be modular, allowing for rapid reconfiguration to work with different body parts.

The ultrasound system may be able to use standard Brightness (B)-mode or harmonic ultrasound imaging, or a variety of other imaging modes, to locate a tumor. Properties of a tumor, such as vascularization, blood flow, or other property variations such as shear stiffness, attenuation, scattering, or appearance, may allow various imaging modalities of the ultrasound system to detect the tumor. For example, these properties may allow detection through imaging modalities such as Doppler flow velocimetry or contrast enhanced relative flow or other methods that measure perfusion, or flow, and can be used to identify areas of increased vascularization that may be associated with the angiogenic nature of tumors. Other imaging modalities used by the ultrasound system may be attenuation imaging, which may leverage wide field matrix arrays, Elastography, which may be used for determining shear stiffness, and plane wave imaging. The ultrasound system may use various different imaging modalities separately or may mix and use different imaging modalities together.

After the ultrasound system has detected a tumor, medical personnel may decide to ablate, or may mark the tumor for follow-up. The decision may also incorporate the suggestion of an artificial intelligence or expert system.

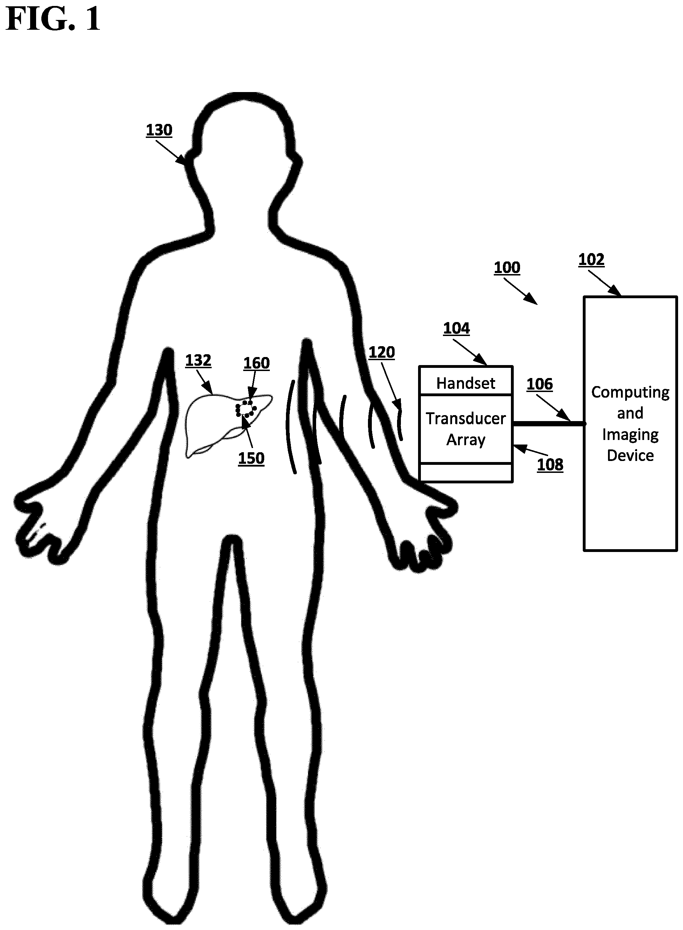

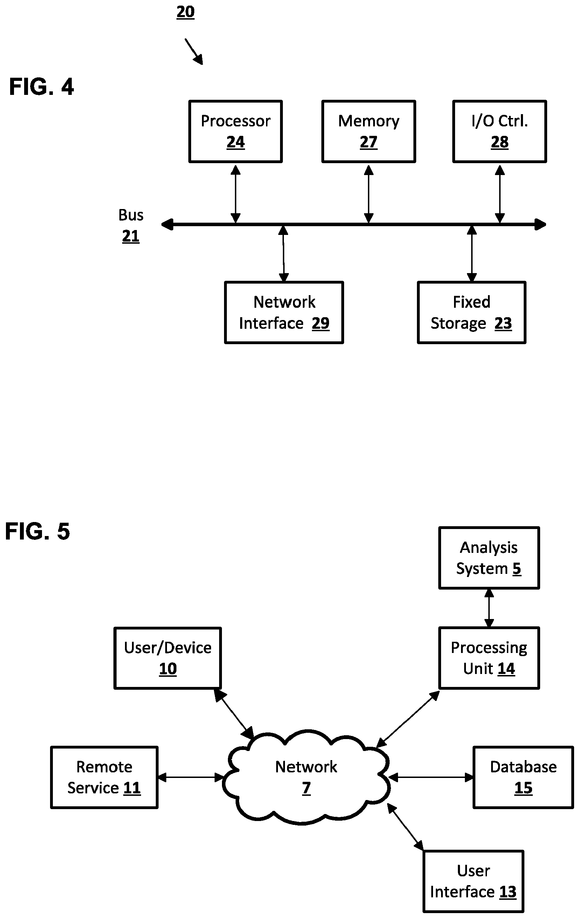

FIG. 1 shows an example arrangement for detection and treatment of tumor using ultrasound. An ultrasonic system 100 may include a handset 104 and a computing and imaging device 102 connected in any suitable manner, such as by a cable 106. The handset 104 may include a transducer array 108 that may include ultrasonic transducer elements arranged in an array. The transducer array 108 may be a large array, for example, with an active area that has a surface area of 15 cm², have an elevation greater than 2 cm, and an azimuth greater than 5 cm. The computing and imaging device 102 may include any suitable computing hardware, running any suitable software, and any other suitable electronics to operate the ultrasonic system 100, including supplying power and control signals to ultrasonic transducer elements of the transducer array 108, for example, through the cable 106, receiving signals from the transducer elements of the transducer array 108, performing any suitable computation to generate images from the signals received from the transducer elements of the transducer array 108, and displaying generated images, for example, on a display directly connection to the computing and imaging device 102, or otherwise sending the generated images to a device, for example, a tablet or phone, that can display the generated images. The computing and imaging device 102 may have any suitable interface to allow a user to control the ultrasound system 100. The computing and imaging device 102 may be, or may include, a computer 20 as showin in FIG. 4.

A patient 130 may undergo any suitable test for early detection of tumors. For example, the test may be a blood test, for example, such as test for blood markers or free circulating DNA, and/or byproduct scans and tests. The results of the test, for example, the free circulating DNA and other data about the tumor determined through the early detection test, may detect the presence of a tumor and may allow the location of the tumor to be narrowed down to a specific organ in the patient 130. For example, the results of the test may indicate that the patient 130 has a tumor 150 in their liver 132.

Moieties 160 may be prepared based on the results of the test and introduced into the patient 130. The moieties 160 may be, for example, tags, or contrast agents, which may have an affinity for tumors and may provide contrast for visibility in images generated by the various imaging modalities. The moieties 160 may be optimized for use with the ultrasound system 100, for example, the moieties 160 may be microbubbles or nanobubbles. The microbubbles or nanobubbles may be filled with a suitable perfluourinated compound. This may allow the micro or nanobubbles to get inside the target cells of the tumor 150 after they are introduced into the patient 130. The moieties 160 may be introduced into the patient 130 in any suitable manner, including, for, example, through injection.

The moieties 160 may be designed to have an affinity for the tumor 150. The affinity of the moieties 160 for the tumor 150 may be based on gross blood flow, for example, based on increased vascularization due to angiogenesis of the tumor 150, or the moieties 160 may be functionalized to attach to surface proteins or other features of the tumor cells of the tumor 150. The moieties 160 may be functionalized based on the surface protein structure of the target cells, or cell surface protein, of the tumor 150. The CSP of the tumor 150 may be found either through wet chemistry of the target cells of the tumor 150 or through cross referencing an extant database that correlates tumors and the CSPs, using data about the tumor obtained, for example, from the blood test or other test type used to detect the presence and determine the location of the tumor 150. The moieties 160 may be functionalized through the use of common streptavidin and biotin complex, or through any other suitable chemistry that may adhere the protein or chemical tags to the moieties 160 and cause them to have an affinity for the tumor 150.

The ultrasound system 100 may include an imaging system, allowing the ultrasound system 100 to operate in an imaging mode that may use any suitable imaging modalities. The transducer array 108 of the ultrasound system 100 may generate ultrasound in the form of ultrasonic waves 120. The handset 104 may positioned so that the transducer array 108 is near, and the ultrasonic waves 120 are targeted at, the liver 132 as the identified location of the tumor 150. For example, the handset 130 may be positioned on the front or back of the patient 130 directly above the liver 132, with the handset 104 near to or in contact with the patient 130, or at any other suitable distance from the patient 130. The transducer array 108 may generate and emit the ultrasonic waves 120 at various frequencies, and may use different frequencies during when operating as an imaging system, including, for example, frequencies that causes the moieties 160, which may be micro or nanobubbles, to heat, expand, oscillate, and/or burst, and imaging ultrasound frequencies that may be used to detect the moieties 160, before or after they are heated, expanded, oscillated, and/or burst in the target cells of the tumor 150.

The transducer array 108 may detect echoes of the ultrasonic waves 120 as they are reflected off of mass on and within the patient 130. Signals generated by the transducer array 108 from detected echoes may be transmitted to the computing and image device 102 of the ultrasound system 100, for example, across the cable 106. The computing and imaging device 102 may use the signals from the transducer array to generate images of the patient 130. The generated images may include the liver 132 and the tumor 150. The generated images may be in the form of live scan slices, or may be, for example, 3D volume images. The 3D volume images may be, for example, voxel images. The transducer array 108 of the ultrasound system 100 may a matrix transducer array, fully sampled in both directions, for example, at least lambda / 2, with no missing elements, and may have a large area, allowing the ultrasound system 100 to generate voxel images with voxels that are smaller in volume than traditional ultrasound systems operating at the same frequency, allowing for higher resolution 3D volume images. The handset 104 and transducer array 108 may not need to be moved during imaging of the liver 150, as the size of the transducer array 150 may allow for the generation of 3D volume images without mechanically scanning the transducer array 108 through movement of the handset 104.

The ultrasound system 100 may be able to use standard B-mode or harmonic ultrasound imaging, or a variety of other imaging modes, to locate the tumor 150 in the patient 130. Properties of the tumor 150, such as vascularization, blood flow, or other property variations such as shear stiffness, attenuation, scattering, or appearance, may allow various imaging modalities of the ultrasound system 100 to detect the tumor 150. For example, these properties may allow detection through imaging modalities such as Doppler flow velocimetry or contrast enhanced relative flow or other methods that measure perfusion, or flow, and can be used to identify areas of increased vascularization that may be associated with the angiogenic nature of the tumor 150. Other imaging modalities used by the ultrasound system 100 may be attenuation imaging, which may leverage wide field matrix arrays, Elastography, which may be used for determining shear stiffness, and plane wave imaging. The ultrasound system 100 may use various different imaging modalities separately or may mix and use different imaging modalities together.

The moieties 160 may also provide information about the path between the tumor 150 and the transducer array 108 of the ultrasound system 100. A nonlinear effect from the moieties 160, for example, micro or nanobubbles, may cause the moieties 160 to radiate energy in different frequency bands. These frequency bands may be used to estimate tissue properties of tissue between the tumor 150 and the transducer array 108, and thus improve resolution and performance of the ultrasound system 100 as a whole.

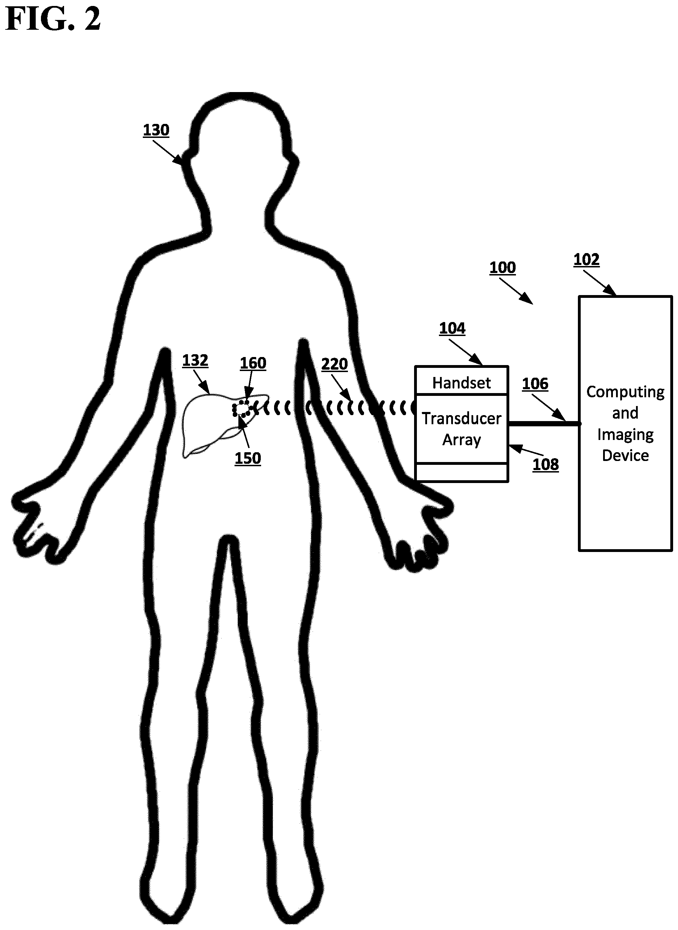

FIG. 2 shows an example arrangement for detection and treatment of tumor using ultrasound. Data resulting from the imaging of the liver 132 by the ultrasound system 100 may be analyzed and presented to a doctor, who may determine an appropriate treatment plan. The data may include, for example, images generated from signals from the transducer array 108 by the computing and imaging device 102, such as 3D volume images that may be voxel images of the liver 132 and the tumor 150, and any data resulting from any suitable analysis of the signals from the transducer array 108. In some implementations, the data resulting from the imaging by the ultrasound system 100 may be input to an artificial intelligence system or expert system that may use the data to suggest an appropriate treatment plan. The treatment plan may identify locations of the liver 132 to which treatment should be applied, for example, to ablate cells at the locations. The identified locations may be based on the locations where the moieties 160 appear in the images resulting from the imaging of the liver 132, as the moieties 160 may be attached to the cells of the tumor 150, allowing for more precise and accurate location of the tumor 150 and any cells or other tumors that may have originated from the tumor 150. The treatment plan may also specify the duration, power level, and frequency of ultrasonic waves to be applied to indicated locations of the liver 132 in order to ablate tissue at the indicated locations that are the targets of the ultrasonic waves.

A treatment plan created based on images of the liver 132 of the patient 130 and data gathered during imaging may identify specific locations of the liver 132 to which treatment should be applied. The transducer array 108 may then be used to apply focused ultrasound to specific locations of the liver 132 identified by the treatment plan with the ultrasound system 100 operating in a therapy mode. For example, the handset 104 may be placed in, or left in, the same position, or a similar position to, the position the handset 104 was placed in when the ultrasound system was operating in an imaging mode to generate the images used to create the treatment plan. The transducer array 108 may be controlled by the computing and imaging device 102, for example, based on input describing the treatment plan, to generate focused ultrasonic waves 220. The focused ultrasonic waves 220 may be generated a power level and frequency that may be capable of ablation of the tumor 150. The focused ultrasonic waves 220 may be targeted in any suitable manner, for example, using any suitable type of beamforming, to be focused on areas of the liver 132 that are indicated for treatment by the treatment plan for durations, and at power levels and frequencies, indicated by the treatment plan. The moieties 160 may be used as a guide to ensure accuracy and precision in the targeting of the focused ultrasonic waves 220.

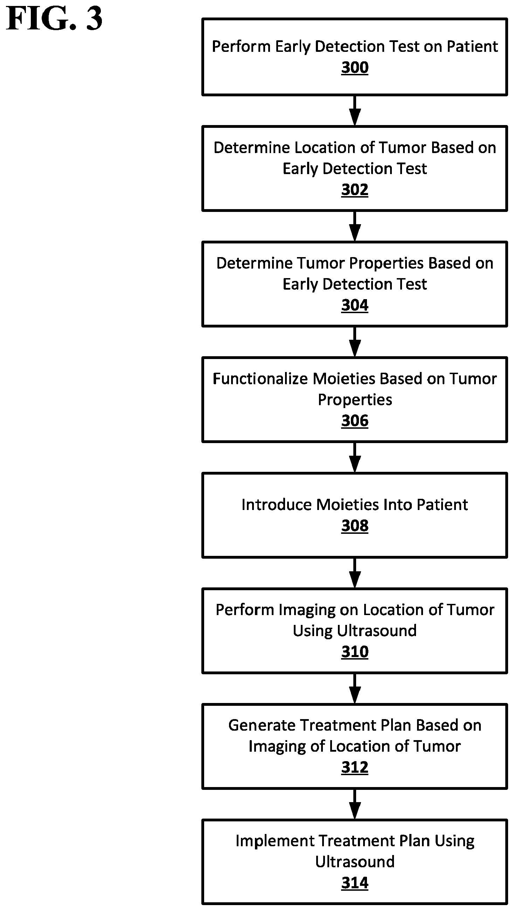

FIG. 3 shows an example procedure for detection and treatment of tumor using ultrasound. At 300, early detection may be performed on a patient. For example, a patient, such as the patient 130, may be given a blood test, such as a liquid biopsy, that may use information such as blood markers and free circulating DNA, and/or byproduct scans and tests, to determine the presence and location of a tumor, such as the tumor 150. The early detection may also be performed using, for example, histology, or using elastography performed using ultrasound, magnetic resonance, or computed tomography.

At 302, the location of a tumor may be determined based on the early detection test. For example, free circulating DNA and other data about the tumor 150 determined based on the early detection performed on the patient 130 may be indicate the type of tumor, which may in turn indicate the likely location of the tumor 150 in the patient 130, for example, in which organ of the patient 130 that tumor 50 is located. The results of the early detection test may, for example, be used to determine that the tumor 150 is located in the liver 132 of the patient 130.

At 304, the properties of a tumor may be determined based on the early detection test. For example, wet chemistry may be performed on target cells of the tumor 150 whose presence was determined by the early detection test to determine the CSP of the tumor 150, or the data about the tumor 150 obtained through the early detection test may be cross referenced against an extant database that correlates tumors and the CSPs. Other properties of the tumor 150 may also be determined.

At 306, moieties may be functionalized based on tumor properties. For example, the moieties 160, before being introduced into the patient 130, may be functionalized based on the properties of the tumor 150 as determined through wet chemistry or cross-referencing against an extant database that correlates tumors and the CSPs, or based on other properties of the tumor 150. The moieties 160 may be functionalized through for example, the use of common streptavidin and biotin complex, or through any other suitable chemistry that may adhere the protein or chemical tags to the moiety, as determined based on the CSP of the tumor 150, allowing the moieties 160 to attach to the CSP or other features of the tumor 150. The moieties may also be functionalized to, for example, target the tumor 150 based on gross blood flow, for example, based on increased vascularization due to angiogenesis of the tumor 150. The moieties 160 may be, for example, microbubbles or nanobubbles filled with a suitable perfluourinated compound, or may include, for example, gadolinium for use with MR imaging systems.

At 308, the moieties may be introduced into the patient. For example, the moieties 160 may be introduced into the patient 130 internally in any suitable manner, such as through injection. Any suitable quantity of the moieties 160 may be introduced into the patient 130.

At 310, imaging on the location of the tumor may be performed using ultrasound. For example, the handset 104 of the ultrasound system 100 may be placed on or near the patient 130 so that the transducer array 108 above the location of the tumor 150, for example, the organ identified as the location of the tumor. For example, the handset 104 may be placed near the liver 132 of the patient 130. The ultrasound system 100, operating in an imaging mode, may image the location of the tumor 150. The ultrasound system 100 may use any suitable imaging modality to image the location of the tumor 150. The transducer array 108 of the ultrasound system 100 may generate ultrasound in the form of ultrasonic waves 120. The handset 104 may positioned so that the transducer array 108 is near, and the ultrasonic waves 120 are targeted at, the liver 132 as the identified location of the tumor 150. The transducer array 108 may generate and emit the ultrasonic waves 120 at various frequencies, and may use different frequencies during when operating as an imaging system, including, for example, frequencies that causes the moieties 160, which may be microbubbles or nanobubbles, to heat, expand, oscillate, and/or burst, and imaging ultrasound frequencies that may be used to detect the heated, expanded, oscillated and/or burst moieties 160, for example, micro or nanobubbles, in the target cells of the tumor 150.

The transducer array 108 may detect echoes of the ultrasonic waves 120 as they are reflected off of mass on and within the patient 130. Signals generated by the transducer array 108 from detected echoes may be transmitted to the computing and image device 102 of the ultrasound system 100, for example, across the cable 106. The computing and imaging device 102 may use the signals from the transducer array to generate images of the patient 130. The generated images may include the liver 132 and the tumor 150. The generated images may be in the form of live scan slices, or may be, for example, 3D volume images. The 3D volume images may be, for example, voxel images. The transducer array 108 of the ultrasound system 100 may a matrix transducer array, fully sampled in both directions, for example, at least lambda / 2, with no missing elements, and may have a large area, allowing the ultrasound system 100 to generate voxel images with voxels that are smaller in volume than traditional ultrasound systems operating at the same frequency, allowing for higher resolution 3D volume images. The handset 104 and transducer array 108 may not need to be moved during imaging of the liver 150, as the size of the transducer array 150 may allow for the generation of 3D volume images without mechanically scanning the transducer array 108 through movement of the handset 104.

The ultrasound system 100 may be able to use standard B-mode or harmonic ultrasound imaging, or a variety of other imaging modes, to locate the tumor 150 in the patient 130. Properties of the tumor 150, such as vascularization, blood flow, or other property variations such as shear stiffness, attenuation, scattering, or appearance, may allow various imaging modalities of the ultrasound system 100 to detect the tumor 150. For example, these properties may allow detection through imaging modalities such as Doppler flow velocimetry or contrast enhanced relative flow or other methods that measure perfusion, or flow, and can be used to identify areas of increased vascularization that may be associated with the angiogenic nature of the tumor 150. Other imaging modalities used by the ultrasound system 100 may be attenuation imaging, which may leverage wide field matrix arrays, Elastography, which may be used for determining shear stiffness, and plane wave imaging. The ultrasound system 100 may use various different imaging modalities separately or may mix and use different imaging modalities together.

In some implementations, magnetic resonance elastography or computed tomography elastography may be used to image the location of the tumor 150 I the patient 130

At 312, a treatment plan may be generated based on the imaging of the location of the tumor. For example, the images of the liver 132 and tumor 150 generated by the ultrasound system 100, and other data gathered during the imaging of the liver 132 and the tumor 150, may be used to generate a treatment plan by, for example, a doctor, artificial intelligence system, or expert system. The treatment plan may, for example, indicate locations of the patient 130 to be targeted with ultrasonic waves, along with durations, frequencies, and power levels for the ultrasonic waves. The locations may be, for example, the location of the tumor 150 as seen in images generated by the ultrasound system 100, as well as locations of any cancerous cells that may have broken from the tumor 150 and have been identified in the images, for example, based on the moieties 160 attaching to the cells. The appearance of the moieties in the images may be used to more accurately and precisely identify the location of the tumor 150 within the liver 132.

At 314, the treatment plan may be implemented using ultrasound. For example, the ultrasound system 100 may operate in a therapy mode. The handset 104 may have been left in position relative to the patient 130 from when the ultrasound system 100 was operating in imaging mode or be replaced to the same position or moved to a different position, as indicated by the treatment plan. The transducer array 108 may be used to apply focused ultrasound to specific areas of the liver 132 identified by the treatment plan with the ultrasound system 100 operating in a therapy mode. For example, the handset 104 may be placed in, or left in, the same position, or a similar position to, the position the handset 104 was placed in when the ultrasound system was operating in an imaging mode to generate the images used to create the treatment plan. The transducer array 108 may be controlled by the computing and imaging device 102, for example, based on input describing the treatment plan, to generate focused ultrasonic waves 220. The focused ultrasonic waves 220 may be generated at a power level and frequency that may be capable of ablation of the tumor 150 and any other cancerous cells identified by imaging of the liver 132. The focused ultrasonic waves 220 may be targeted in any suitable manner, for example, using any suitable type of beamforming, to be focused on areas of the liver 150 that are indicated for treatment by the treatment plan for durations, and at power levels and frequencies, indicated by the treatment plan. The moieties 160 may be used as a guide to ensure accuracy and precision in the targeting of the focused ultrasonic waves 220.

In some implementations, treatment according to the treatment plan may be implemented with a system different from that used to image the location of the tumor 150. For example, an ultrasound system that is different from the ultrasound system 100 may be used for one of the imaging and treatment, or the ultrasound system 100 may be used for treatment when a system for magnetic resonance or computed tomography was used for imaging the location of the tumor 150.

Embodiments of the presently disclosed subject matter may be implemented in and used with a variety of component and network architectures. FIG. 4 is an example computer system 20 suitable for implementing embodiments of the presently disclosed subject matter. The computer 20 includes a bus 21 which interconnects major components of the computer 20, such as one or more processors 24, memory 27 such as RAM, ROM, flash RAM, or the like, an input/output controller 28, and fixed storage 23 such as a hard drive, flash storage, SAN device, or the like. It will be understood that other components may or may not be included, such as a user display such as a display screen via a display adapter, user input interfaces such as controllers and associated user input devices such as a keyboard, mouse, touchscreen, or the like, and other components known in the art to use in or in conjunction with general-purpose computing systems.

The bus 21 allows data communication between the central processor 24 and the memory 27. The RAM is generally the main memory into which the operating system and application programs are loaded. The ROM or flash memory can contain, among other code, the Basic Input-Output system (BIOS) which controls basic hardware operation such as the interaction with peripheral components. Applications resident with the computer 20 are generally stored on and accessed via a computer readable medium, such as the fixed storage 23 and/or the memory 27, an optical drive, external storage mechanism, or the like.

Each component shown may be integral with the computer 20 or may be separate and accessed through other interfaces. Other interfaces, such as a network interface 29, may provide a connection to remote systems and devices via a telephone link, wired or wireless local- or wide-area network connection, proprietary network connections, or the like. For example, the network interface 29 may allow the computer to communicate with other computers via one or more local, wide-area, or other networks, as shown in FIG. 5.

Many other devices or components (not shown) may be connected in a similar manner, such as document scanners, digital cameras, auxiliary, supplemental, or backup systems, or the like. Conversely, all of the components shown in FIG. 4 need not be present to practice the present disclosure. The components can be interconnected in different ways from that shown. The operation of a computer such as that shown in FIG. 4 is readily known in the art and is not discussed in detail in this application. Code to implement the present disclosure can be stored in computer-readable storage media such as one or more of the memory 27, fixed storage 23, remote storage locations, or any other storage mechanism known in the art.

FIG. 5 shows an example arrangement according to an embodiment of the disclosed subject matter. One or more clients 10, 11, such as local computers, smart phones, tablet computing devices, remote services, and the like may connect to other devices via one or more networks 7. The network may be a local network, wide-area network, the Internet, or any other suitable communication network or networks, and may be implemented on any suitable platform including wired and/or wireless networks. The clients 10, 11 may communicate with one or more computer systems, such as processing units 14, databases 15, and user interface systems 13. In some cases, clients 10, 11 may communicate with a user interface system 13, which may provide access to one or more other systems such as a database table 15, a processing unit 14, or the like. For example, the user interface 13 may be a user-accessible web page that provides data from one or more other computer systems. The user interface 13 may provide different interfaces to different clients, such as where a human-readable web page is provided to web browser clients 10, and a computer-readable API or other interface is provided to remote service clients 11. The user interface 13, database table 15, and processing units 14 may be part of an integral system, or may include multiple computer systems communicating via a private network, the Internet, or any other suitable network. Processing units 14 may be, for example, part of a distributed system such as a cloud-based computing system, search engine, content delivery system, or the like, which may also include or communicate with a database table 15 and/or user interface 13. In some arrangements, an analysis system 5 may provide back-end processing, such as where stored or acquired data is pre-processed by the analysis system 5 before delivery to the processing unit 14, database table 15, and/or user interface 13. For example, a machine learning system 5 may provide various prediction models, data analysis, or the like to one or more other systems 13, 14, 15.

This application further discloses:
1. A method for detection and treatment of tumors using ultrasound comprising:
   performing an early detection test on a patient;
   determining a location of a tumor based on the early detection test;
   determining one or more properties of the tumor based on the early detection test;
   functionalizing moieties based on the properties of the tumor; introducing the moieties into the patient;
   imaging the location of the tumor using one or more of ultrasound, magnetic resonance elastography, and computed tomography elastography, to generate one or more images of the location of the tumor; and
   implementing a treatment plan, the treatment plan based on the one or more images of the location of the tumor, using ultrasound.
2. The method of item 1, wherein the early detection test is a blood test, a histology test, or elastography.
3. The method of item 1 or 2, wherein determining one or more properties of the tumor based on the early detection test further comprises identifying a cell surface protein (CSP) of the tumor.
4. The method of item 1, 2, or 3, wherein functionalizing moieties based on the properties of the tumor further comprises one or more of using common streptavidin and biotin complex, using chemistry to adhere protein or chemical tags to the moieties based on a CSP of the tumor, and causing the moieties to target the tumor based on gross blood flow to the tumor.
5. The method of one of items 1 to 4, wherein the moieties are microbubbles or nanobubbles filled with perfluourinated compound, or the moieties comprise gadolinium.
6. The method of one of items 1 to 5, wherein imaging the location of the tumor using ultrasound to generate one or more images of the location of the tumor further comprises generating 3D volume images of the location of the tumor based on ultrasonic waves generated by a transducer array of an ultrasound system.
7. The method of one of items 1 to 6, wherein imaging the location of the tumor using ultrasound to generate one or more images of the location of the tumor further comprises generating ultrasonic waves at a frequency that causes the moieties to one or more of heat, expand, oscillate, and burst.
8. The method of one of items 1 to 7, wherein the ultrasound is generated by a transducer array that has an active area with a surface area of greater than 15 square centimeters.
9. The method of one of items 1 to 8, wherein implementing a treatment plan, the treatment plan based on the one or more images of the location of the tumor, using ultrasound further comprises generating ultrasound waves targeted to locations identified by the treatment plan using a transducer array of an ultrasound system.
10. The method of one of items 1, wherein the treatment plan comprises indications of locations to which ultrasound should be applied and durations, frequencies, and power levels at which ultrasound should be applied to the indicated locations.
11. The method of item 10, wherein the indications of locations to which ultrasound should be applied are based on the appearance of the moieties in the images of the location of the tumor.
12. The method of one of items 1 to 11, wherein the images of the location of the tumor are voxel images.
13. The method of one of items 1 to 12, wherein imaging the location of the tumor using ultrasound to generate one or more images of the location of the tumor and implementing a treatment plan, the treatment plan based on the one or more images of the location of the tumor, using ultrasound use a same transducer array, and further comprising:
   not moving the transducer array during both of, and between, the imaging of the location of the tumor and the implementing of the treatment plan.
14. The method of one of items 1 to 13, wherein imaging the location of the tumor using ultrasound to generate one or more images of the location of the tumor further comprises performing, with an ultrasound system, one or more of Doppler flow velocimetry, contrast enhanced relative flow, perfusion measurement, attenuation imaging, elastography, brightness mode imaging, harmonic ultrasound imaging, and plane wave imaging.
15. A method for detection and treatment of tumors using ultrasound comprising:functionalizing moieties to attach to cells of a tumor based on properties of the tumor, wherein the moieties act as contrast agents;introducing the moieties into the patient after the moieties are functionalized;
   imaging a location of the tumor using ultrasonic waves generated by an ultrasound system, or using magnetic resonance system, to generate one or more images of the location of the tumor, the one or more images comprising images of the moieties attached to one or more cells of the tumor; and applying therapy to locations of the moieties in the patient, wherein the therapy comprises ultrasonic waves generated by the ultrasound system.
16. The method of item 15, wherein the properties of the tumor are determined based on one or more of a blood test, a histology test, and elastography.
17. The method of item 15 or 16, wherein applying therapy to locations of the moieties comprises targeting the ultrasonic waves at one or more of the locations of the moieties, wherein the one or more locations are indicated by a treatment plan based on the images of the location of the tumor.
18. The method of item 15, 16, or 17 wherein the one or more images of the location of the tumor are 3D volume images.
19. The method of one of items 15 to 18, further comprising, while imaging a location of the tumor using ultrasound to generate one or more images of the location of the tumor, the one or more images comprising images of the moieties attached to one or more cells of the tumor, generating with an ultrasound system ultrasonic waves at one or more frequencies that cause the moieties to one or more of heat, expand, oscillate, and burst.
20. The method of one of items 15 to 19, wherein the ultrasonic waves generated by the ultrasound system are generated by a transducer array of the ultrasound system that has an active area with a surface area greater than 15 cm2.

The foregoing description, for purpose of explanation, has been described with reference to specific embodiments. However, the illustrative discussions above are not intended to be exhaustive or to limit embodiments of the disclosed subject matter to the precise forms disclosed. Many modifications and variations are possible in view of the above teachings. The embodiments were chosen and described in order to explain the principles of embodiments of the disclosed subject matter and their practical applications, to thereby enable others skilled in the art to utilize those embodiments as well as various embodiments with various modifications as may be suited to the particular use contemplated.

## Claims

1. A method for detection of tumors using ultrasound comprising:
functionalizing moieties to attach to cells of a tumor based on properties of the tumor, wherein the moieties act as contrast agents;
introducing the moieties into the patient after the moieties are functionalized;
and imaging a location of the tumor using ultrasonic waves generated by an ultrasound system, or using magnetic resonance system, to generate one or more images of the location of the tumor, the one or more images comprising images of the moieties attached to one or more cells of the tumor.

2. The method of claim 1, wherein the properties of the tumor are determined based on one or more of a blood test, a histology test, and elastography.

3. The method of claim 1 or 2, further comprising, while imaging a location of the tumor using ultrasound to generate one or more images of the location of the tumor, the one or more images comprising images of the moieties attached to one or more cells of the tumor, generating with an ultrasound system ultrasonic waves at one or more frequencies that cause the moieties to one or more of heat, expand, oscillate, and burst.

4. The method for detection of tumors using ultrasound of claim 1, further comprising:
performing an early detection test on a patient;
determining a location of a tumor based on the early detection test;
determining one or more properties of the tumor based on the early detection test; and
functionalizing the moieties based on the properties of the tumor.

5. The method of claim 4, wherein the early detection test is a blood test, a histology test, or elastography.

6. The method of claim 4 or 5, wherein determining one or more properties of the tumor based on the early detection test further comprises identifying a cell surface protein (CSP) of the tumor.

7. The method of claim one of claims 4 to 6, wherein functionalizing moieties based on the properties of the tumor further comprises one or more of using common streptavidin and biotin complex, using chemistry to adhere protein or chemical tags to the moieties based on a CSP of the tumor, and causing the moieties to target the tumor based on gross blood flow to the tumor.

8. The method of one of claims 1 to 7, wherein the moieties are microbubbles or nanobubbles filled with perfluourinated compound, or the moieties comprise gadolinium.

9. The method of one of claims 1 to 8, wherein imaging the location of the tumor using ultrasound to generate one or more images of the location of the tumor further comprises generating 3D volume images of the location of the tumor based on ultrasonic waves generated by a transducer array of an ultrasound system.

10. The method of one of claims 1 to 9, wherein the ultrasound is generated by a transducer array that has an active area with a surface area of greater than 15 square centimeters.

11. The method of claim 9 or 10, further comprising:
not moving the transducer array during the imaging of the location of the tumor and before implementing a treatment plan using ultrasound emitted by the same transducer array.

12. The method of one of claims 1 to 11, wherein imaging the location of the tumor using ultrasound to generate one or more images of the location of the tumor further comprises generating ultrasonic waves at a frequency that causes the moieties to one or more of heat, expand, oscillate, and burst.

13. The method of one of claims 1 to 12, wherein the indications of locations to which ultrasound should be applied are based on the appearance of the moieties in the images of the location of the tumor.

14. The method of one of claims 1 to 13, wherein the images of the location of the tumor are voxel images.

15. The method of one of claims 1 to 14, wherein imaging the location of the tumor using ultrasound to generate one or more images of the location of the tumor further comprises performing, with an ultrasound system, one or more of Doppler flow velocimetry, contrast enhanced relative flow, perfusion measurement, attenuation imaging, elastography, brightness mode imaging, harmonic ultrasound imaging, and plane wave imaging.
